Europäisches Patentamt

(19) European Patent Office

Office européen des brevets

(11) **EP 0 499 456 B1**

(12) **EUROPEAN PATENT SPECIFICATION**

(45) Date of publication and mention
of the grant of the patent:
**04.12.1996 Bulletin 1996/49**

(51) Int. Cl.$^6$: **C07C 229/08**, C07F 7/00,
A61K 7/38, A61K 7/34

(21) Application number: **92301158.9**

(22) Date of filing: **12.02.1992**

(54) **Zirconium/aluminum antiperspirant material and anti-perspirant compositions and products containing same**

Zirkonium/Aluminium Antischwitzenstoff und antischwitzende Zusammensetzungen und diese enthaltende Produkt

Matériel antiperspirant de Zirconium/Aluminium et composition antiperspirant et produits qui les contiennent

(84) Designated Contracting States:
**DE FR GB GR IT SE**

(30) Priority: **13.02.1991 US 654480**

(43) Date of publication of application:
**19.08.1992 Bulletin 1992/34**

(73) Proprietor: **Bristol-Myers Squibb Company**
**New York, N.Y. 10154 (US)**

(72) Inventors:
• **Shin, Chung T.**
**Livington, New Jersey 07039 (US)**
• **Rosenberg, Allan H.**
**South Orange, New Jersey 07079 (US)**

• **Markarian, Herand M.**
**Congers, New York 10920 (US)**

(74) Representative: **Cropp, John Anthony David**
**MATHYS & SQUIRE**
**100 Grays Inn Road**
**London, WC1X 8AL (GB)**

(56) References cited:
**EP-A- 0 047 650**          **FR-A- 2 495 933**

Remarks:
The file contains technical information submitted after the application was filed and not included in this specification

EP 0 499 456 B1

## Description

This invention relates to zirconium-aluminum-amino acid salts, the preparation of the salts and of aqueous solutions thereof, to antiperspirant compositions containing the salts, to processes for preparing the compositions and to antiperspirant products containing the compositions.

The preparation of aluminum- and zirconium-based antiperspirant materials generally involves combining compounds of these metals with an amino acid under suitable conditions. Such materials are generally referred to as ZAG complexes and have often been shown to be chemically distinct species having antiperspirant efficacy, i.e., the inhibition of perspiration when applied to human skin, especially the axilla.

In U.S. Patent 4,775,528 to Callaghan et al, an antiperspirant composition is produced by heating a 2 to 18% aqueous solution of aluminum chlorhydroxide to at least 50°C, mixing zirconyl hydroxychloride with the solution before, during or after heating, continuing heating of the aqueous mixture until prescribed characterizations are achieved, and thereafter rapidly drying the heated aqueous mixture to obtain a solid material having an Al:Zr atomic ratio of from 6:1 to 1:1 and also having peak 4 and peak 3 Al chromatograph peaks, the ratio of peak 4 to peak 3 being at least 2:1. Patentees specify in Claim 1 that the zirconyl hydroxychloride be mixed with the aluminum chlorhydroxide solution before the solution loses all of its water, i.e., "before completion of . . . drying".

U.S. 4,818,512 to Markarian et al -- which is assigned to applicants' assignee -- describes the production of an improved activated aluminum chlorhydroxide material from a conventional activated aluminum chlorhydroxide material, the improved material being characterized by containing a high concentration (77 to 85% by weight) of the $K_d$ 0.4 fraction. The improved aluminum chlorhydroxide of the '512 patent is prepared by heating a 5% aqueous solution of the conventional aluminum chlorhydroxide material under certain conditions and spray-drying the product. The material made in the '512 patent may be combined with zirconium-containing species to yield zirconium-aluminum-glycine (ZAG) complexes or compounds, which are highly efficacious as antiperspirants.

EP-A-47650 describes an antiperspirant complex comprising an aluminum chlorhydroxide, a zirconium hydroxychloride, a water soluble neutral amino acid and an inorganic acid compound. The molar ratio of aluminium to zirconium is required to be in the range 2:1 to 10:1.

It has been discovered that, under certain conditions, solutions of certain aluminum chloro species (as hereinafter described) and zirconium hydroxychloride, i.e., $ZrO(OH)Cl$, can be admixed in the presence of an amino acid, as to yield a ZAG material, the solid form of the material being obtained by conventional drying means, such as by steam bath evaporation, spray-drying and freeze-drying. The ZAG material is believed to be a new chemical entity having a unique chemical formula. Antiperspirant compositions having superior antiperspirant properties are obtained when the ZAG material is formulated with activated aluminum chlorhydroxide, $Al_2(OH)_5Cl$ (hereinafter also referred to as "ACH"), as hereinafter described.

This new material is a zirconium-aluminum-glycine (ZAG) or Al/Zr polychlorohydrex-glycine material, which is referred to herein variously as a "ZAG compound", "ZAG material", "ZAG complex" or "ZAG salt". For simplicity, applicants will generally refer to the dried ZAG material as a ZAG salt. The term "ZAG complex" is often used by practitioners of this art, but in a general sense, since the mechanism by which the ZAG material is formed is not well understood. The ZAG material is a complex in the sense that the various constituents interact in a chemical or physical manner to provide the subject ZAG species.

By "aluminum chloro species" applicants mean aluminum chloride and aluminum hydroxy chloro species defined by the formula $Al_2(OH)_{6-x}Cl_x$ wherein $X = 1.5$ to 6, in particular, 2/3 basic aluminum chloride, $Al_2(OH)_4Cl_2$, and 3/4 basic aluminum chloride, $Al_2(OH)_{4.5}Cl_{1.5}$. Thus, when $x = 6$ the salt is aluminum chloride, $AlCl_3$, which salt may be hydrated with up to 6 moles water.

In this specification and in the claims, unless otherwise indicated, all percents are percent by weight and all parts are parts by weight.

Thus, according to the present invention there is provided a zirconium-aluminum-amino acid (ZAG) material containing on a weight basis: 23.0-34.7wt.% Zr, 1.7-2.5wt.% Al, 21.3-31.9wt.% amino acid and 15.5-23.3wt.% chloride, said material being bound with zero to nine moles water per mole of the material, and obtained by a process comprising the steps of

admixing zirconium hydroxychloride, an aluminum chloro species of the formula $Al_2(OH)_{6-x}Cl_x$ wherein $x = 1.5$ to 6 and an amino acid in an aqueous solution at from 21 to 116°C (70 to 240°F), and
drying the aqueous solution.

The invention also provides a process for producing a zirconium-aluminum-amino acid material containing on a weight basis: 23.0-34.7 wt.% Zr, 1.7-2.5 wt.% Al, 21.3-31.9 wt.% amino acid and 15.5-23.3 wt.% chloride, said material being bound with zero to nine moles water per mole of the material, said process comprising the steps of

(a) admixing zirconium hydroxychloride, an aluminum chloro species of the formula $Al_2(OH)_{6-x}Cl_x$ wherein $x = 1.5$

to 6 and an amino acid in an aqueous solution at from 21 to 116°C (70 to 240°F), and
drying the aqueous solution.

The invention further provides a solid antiperspirant composition comprising a zirconium-aluminum-amino acid (ZAG) material as defined above and an activated aluminum chlorhydroxide, more than 40% of the Al peaks as determined by gel chromatography of the antiperspirant composition having a $K_d$ value of 0.4.

The antiperspirant composition may be obtained by dry-blending the ZAG salt of the present invention and an activated aluminum chlorhydroxide or, alternatively, by mixing a solution of the ZAG material and a solution of an activated aluminum chlorhydroxide and then rapidly drying same. As used herein, "antiperspirant product" means the antiperspirant composition incorporated into a cosmetically acceptable carrier or vehicle, as hereinafter described.

In a preferred embodiment of the process described above, 66.8 parts of a nominal 50% ZrO(OH)Cl solution, 19.4 parts of a 50% $AlCl_3$ solution and 13.8 parts of glycine were heated together on a steam bath and dried, to yield a dry powder (ZAG salt) containing on assay 26.6% zirconium, 2.0% aluminum, 24.4% glycine (percent glycine calculated by difference) and 18.1% chloride. A ZAG salt containing 25.5% Zr, 1.9% Al, 23.6% glycine and 17.5% chloride --which values are only slightly less than the assayed ZAG salt made by the described procedure -- has an empirical formula of

$$AlZr_4O_4(OH)_4Cl_7Gly_{4.5} \cdot nH_2O$$

wherein n = 0-9, preferably 2-7.

An antiperspirant composition was obtained by dry-blending 48 parts of the above-described ZAG salt with 52 parts ACH powder. The resulting antiperspirant composition contained 13.3% Al, 12.4% Zr and 16.8% Cl and had an Al/Zr atomic ratio of 3.6:1 and a metal/chloride ratio of 1.3:1.

Similarly, a solution containing 66.8 parts of a nominal 50% ZrO(OH)Cl solution, 19.4 parts of a 50% $AlCl_3$ solution and 13.8 parts glycine was spray-dried to yield a ZAG salt containing on assay 28.7% Zr, 2.1% Al, 26.6% glycine and 19.4% chloride. The empirical formula was similar to that given above. This ZAG salt was blended or mixed in a dry state with dry ACH to yield a highly useful antiperspirant composition.

In another embodiment of the present invention, the antiperspirant composition is made by first preparing an aqueous solution of ZrO(OH)Cl, the aluminum chloro species and the amino acid, preferably by mixing the solution at about room temperature to obtain the ZAG material, combining said solution containing the ZAG material with a solution of ACH preferably at about room temperature, and thereafter rapidly drying the solution.

Advantages

The compounds and compositions of the invention have several advantages over other antiperspirants. The ZAG/ACH combinations made in accordance with the invention are significantly more efficacious than are commercially available tetra ZAG salts.

In addition, the ZAG salts made in accordance with the process of the invention are characterized by their

(1) Ease of manufacture,
(2) More uniform chemical properties, and
(3) Good stability during the mixing process.

These and other advantages and aspects of the invention will be apparent after a consideration of the following description and claims.

Detailed Description of the Invention

The description of the invention will focus on discussions of the new ZAG salt, the ACH with which it is combined, and the final antiperspirant compositions.

ZAG Ingredient (Component I)

The new ZAG compounds of the present invention are aluminum-zirconium polychlorohydrex-amino acid materials. Glycine is the preferred amino acid. The description herein shall refer, for simplicity, to glycine when generally describing the amino acid, except when the context indicates otherwise, as in the illustrative examples and specific embodiments.

In the past Al-Zr-glycine complexes have been made by heating an aqueous solution of aluminum chlorohydrate with zirconium and glycine for appropriate periods of time. Previously cited U.S. Patent 4,775,528 to Callaghan et al disclosed such a process.

The ZAG complexes of the present invention are polymeric species which have proportions of zirconium, aluminum, glycine and chloride as set forth in Table I. The atomic ratio of Zr:Al is in the range of about 6:1 to about 2.75:1.

The ZAG complexes of the present invention are made by preparing an aqueous solution of zirconium hydroxychloride (ZrO(OH)Cl); an aluminum chloro species; an amino acid, for example, glycine, lycine and alanine and heating the solution. The aqueous solution suitably contains from 25 to 40%, preferably from 30 to 37% ZrO(OH)Cl, from 8 to 12%, preferably from 9 to 11% $AlCl_3$, and from 11 to 17%, preferably from 12 to 15% amino acid.

The individual components may be added as dry powders to water to form the aqueous solution. Preferably, premixes of ZrO(OH)Cl and $AlCl_3$ are employed, with amino acid being added to either premix or to the combined premix solutions.

Commercially available ZrO(OH)Cl solutions (i.e., 25 to 50% aqueous ZrO(OH)Cl are especially useful. Basic zirconyl chloride or zirconyl hydroxychloride available from Westwood Chemical (Middletown, New York) and Magnesium Electron, Inc. can be used. Mixtures are operable. As previously described, the aluminum chloro species component of the ZAG material is a salt of the general formula $Al_2(OH)_{6-x}Cl_x$ wherein x = 1.5-6, for example, $AlCl_3$, 2/3 and 3/4 basic aluminum chlorides. The aluminum chloro species are typically available commercially and used as aqueous solutions. Aluminum chloride is the preferred aluminum chloro species, as this salt is the most acidic of those included in the general formula. Aqueous aluminum chloride solutions having a 50% actives concentration are available from Reheis Chemical Co. These commercially available zirconium and aluminum salt solutions are provided as "nominal" solutions, e.g., a 50% solution contains approximately 50% of the active ingredient.

The zirconium-containing solution is combined with the aluminum chloride solution via simple mixing at a temperature in the range of from 21 up to 116°C (70 up to 240°F) preferably from 21 to 60°C (70 to 140°F). Generally, mixing time is not critical, and is completed in under 15 minutes, preferably within 10 minutes, the time required for mixing depending on the size, temperature and degree of agitation of the batch. Longer mixing times are possible, but are not economically practical. Heating of the solution when elevated temperature is used may be conducted by conventional means such as a steam bath, steam jacket and closed loop circulation through a heat exchanger. At higher temperatures system pressures would be such as to avoid boiling of the aqueous solution and to ensure suitable control of the process. The pressure can be determined easily by using steam tables. It is believed that the ZAG complex is formed during this mixing procedure. However, applicants do not wish to be bound to any particular theory regarding complexation or salt formation.

The third component is a commercial amino acid typically of 99 to 100 percent purity, preferably glycine. Suitable glycines are glycine crystals made by Robeco Company of New York and glycine powders made by Chattem Company of Tennessee. Other amino acids, e.g., alanine and the like, are also suitable.

It is convenient to form aqueous premixes as of the ZrO(OH)Cl and the aluminum chloro constituents, and thereafter to combine these solutions, along with the glycine component. The glycine component may be added to either premix or to the combined premix solutions. An aqueous premix of the glycine component is also acceptable. Alternatively, an aqueous solution of ZrO(OH)Cl and the aluminum chloro species can be prepared based on the use of the dry materials, although this is not preferred. Also, any one of the dry components can be added to an aqueous premix of another component.

As previously mentioned, aqueous solutions of ZrO(OH)Cl and the aluminum chloro species are commercially available and may be used as the premix solutions with or without dilution. The use of premix solutions is preferred for ease of handling.

After completion of mixing, the ZAG salt, i.e., the dry ZAG complex, can be obtained by drying the solution to substantial dryness. The time to effect drying is not critical, and will depend on the size of the batch, the temperature and pressure of the mixing vessel, and the drying equipment used. It is preferable to effect drying rapidly, which can be accomplished best by spray-drying techniques well known in the art. A portion of the water in the aqueous solution may evaporate during the mixing step when elevated temperatures are used. Drying methods include those which employ a steam bath, spray-drying, freeze-drying, etc. Drying is carried out until an essentially powdery state is reached.

The process described above may be conducted by batch, semi-batch or continuous techniques. Typically, a semi-batch process is employed with preparation of a given quantity of the solution mixture containing the components, with direct feed to a spray drying chamber. In this case drying is rapid, especially when elevated temperatures are used. The solution mixture may be prepared by simultaneously charging the mixture vessel with premix solutions.

Once the powder has been obtained, it may be ground (e.g., via jet-milling or other commercial process) to yield an impalpable powder for storage, shipment, or further processing. Tipically, 97% of this powder passes through a 325 mesh screen, and provides good organoleptic properties when provided in an antiperspirant product.

Table I sets forth a typical assay of the ZAG salt (Component I) obtained by the process of the present invention.

TABLE I

| COMPOSITION OF ZAG INGREDIENT (COMPONENT I) | | | |
|---|---|---|---|
| | Broad Range | Preferred Range | Highly Preferred |
| % Zr | 23.0-34.7 | 25.8-31.6 | 28.7 |
| % Al | 1.7- 2.5 | 1.9- 2.3 | 2.1 |
| % Amino Acid | 21.3-31.9 | 23.9-29.2 | 26.6 |
| % Chloride | 15.5-23.3 | 17.5-21.3 | 19.4 |

While the exact nature of the ZAG salt is not readily ascertained, the ZAG salt, based on a theoretical correlation of the assays made on the material, generally conforms to the empirical formula

$$AlZr_4O_4(OH)_4Cl_7Gly_{4.5} \cdot nH_2O$$

wherein n = 0 to 9.

Activated ACH (Component II)

The ZAG compound may be employed to form an improved antiperspirant composition also containing an activated aluminum chlorhydroxide (ACH) component. ACH components are well known in the art, as described, for example, in Antiperspirants and Deodorants, K. Laden (Editor), pp. 214-8 (1989). See also U.S. 3,904,741 to Jones et al.

One highly useful ACH is described in the previously cited U.S. Patent 4,818,512 to Markarian et al.

The preferred ACH component of the antiperspirant composition typically has the following approximate distribution for the Al species:

TABLE II

| PERCENT ALUMINIUM PEAK HEIGHTS FOR ACH | | | | |
|---|---|---|---|---|
| | $K_d = 0$ | $K_d = 0.25$ | $K_d = 0.4$ | $K_d = 0.6$ |
| Preferred Range | 0-3 | 25-35 | 50-60 | 5-20 |

Commercially available ACH powders are available as the products sold under the trade names DM200 or DM250 by Westwood Chemical (Middletown, New York), RE101 and RE103 by Reheis (Berkely, New Jersey) and Q5-7171 (AAH) by Dow Corning (Midland, Michigan).

Antiperspirant (ZAG/ACH) Combination Compositions

The ZAG salt and the ACH components, discussed above, may be combined, undiluted with carrier(s), via simple mixing in a substantially dry state to obtain a homogeneous blend, typically for between 30 to 120 minutes, preferably for between 60 to 90 minutes, to yield a highly efficacious antiperspirant composition. The weight ratio of ZAG salt to ACH present in the antiperspirant composition is from 2:1 to 1:2, preferably from 1.5:1 to 1:1.5, most preferably 1:1. A typical antiperspirant composition contains 13.3% Al, 12.4% Zr, 16.8% Cl and 11.5% glycine, and has an Al/Zr atomic ratio of about 3.6:1 and a metal/chloride atomic ratio of 1.3:1.

In another embodiment of the present invention, the antiperspirant composition is prepared by first forming the aqueous solution containing the ZrO(OH)Cl, the aluminum chloro species and the glycine constituents, combining said solution with the activated aluminum chlorhydroxide, and rapidly thereafter drying said solution to form the solid antiperspirant composition. In this embodiment it is important that the mixing of the constituents be conducted at less than 52°C (125°F), and preferably at ambient. The weight ratio of the ZAG salt to the ACH present in the antiperspirant composition is as previously described. The assay and Al/Zr and metal/chloride atomic ratios for the antiperspirant composition in accordance with this process embodiment are also as previously described.

In the process described in the paragraph above, the time in which the ACH and the ZAG material are present together in the in the aqueous phase should not be excessive, in order to obtain a final antiperspirant composition having a high proportion of $K_d$ 0.4 for the aluminum species. Accordingly, the preparation of the combined ZAG-ACH solution and the drying process should both preferably be effected rapidly, within the practical time limits imposed by conventional processing equipment. The combined ZAG complex-ACH solution is typically ready for processing in the drying equipment as soon as an essentially homogeneous mixture is obtained. Generally, this is within about 5 to 20 minutes, preferably within 5 minutes. Drying should also be completed rapidly. In general, using a shorter mixing residence time provides greater leeway for the time in which to carry out the drying procedure. Spray-drying provides essentially instantaneous drying, and accordingly is highly preferred. The mixing and drying steps, in total, should not exceed about 30 minutes, and preferably are effected in less than about 15 minutes.

The final dry antiperspirant composition obtained in accordance with the embodiments of the present invention was analyzed using the gel chromatographic technique set forth below. The ZAG/ACH antiperspirant composition of the invention has the following Al species $K_d$ distribution for the Al species:

TABLE III

| % Al $K_d$ PEAK HEIGHTS FOR ZAG/ACH COMBINATIONS | | | | |
|---|---|---|---|---|
| | $K_d$ = 0 | $K_d$ = 0.25 | $K_d$ = 0.4 | $K_d$ = 0.6 |
| Broad Range | 0-5 | 30-40 | 40-60 | 5-20 |
| Preferred Range | 0-3 | 25-35 | 50-60 | 5-20 |

In general, combinations in which $K_d$ 0.4 for 50% or more of the Al peaks are preferred.

Polymer Characterization Techniques

The character of the ACH and of the ZAG/ACH compositions was determined using the following procedure.

Aluminum species distribution for ACH and ZAG/ACH mixtures are obtained from gel filtration chromatography. The principles of gel filtration are discussed in Gel Filtration Theory and Practice published by Pharmacia Fine Chemicals, Uppsala, Sweden (November 1981-82).

As explained in Gel Filtration Theory and Practice, $K_d$ is the distribution coefficient, which is defined by the equation

$$K_d = (V_e - V_o)/V_s$$

where

$V_e$ = elution volume
$V_o$ = void volume
$V_s$ = volume of the stationary phase

Generally, the gel chromatograph measures $K_{av}$, defined by the equation

$$K_{av} = (V_e - V_o)/(V_t - V_o)$$

where

$V_t$ = total volume

$K_{av}$ represents the fraction of the stationery gel volume which is available for diffusion of a given solute species. For a given gel, there is a constant ratio $K_{av}:K_d$ which is independent of the nature of the solute or its concentration. In the characterizations of the active antiperspirant components and compositions, the gel chromatography technique measures $K_{av}$. Measurements using the gel chromatography techniques herein described and used in the characterization of the antiperspirant actives, in fact, measure the parameter $K_{av}$. However, because the ratio $K_{av}:K_d$ is close to unity, it is common practice in the antiperspirant art to use these parameters interchangeably.

Metal analysis of the separated species eluting off the column are obtained by ICP spectroscopy. The following conditions are employed.

Column        : Sephadex G-25 resin (superfine) 50 cm manufactured by Pharmacia Fine Chemicals
Eluent        : 0.2M KCl/0.0035 M HCl
Sample Size   : 100 $\mu$l of a freshly prepared 13% aqueous solution of activated ACH or ZAG/ACH antiperspirant composition

Flow Rate       : 0.4 ml/min.
RI Detector      : Shimatzu RID 6A
Metal Analyzer  : Perkin Elmer ICP 6500

Other Ingredients

The ZAG salt and the antiperspirant compositions of the present invention may be incorporated into a cosmetically acceptable carrier or vehicle to provide commercially useful antiperspirant products.

The antiperspirant products of the invention may contain a wide variety of materials conventionally employed in the manufacture of such products.

Excipients such as fillers, diluents, coupling agents, pH modifiers, buffers, colorants (e.g., opacifiers, whiteners), perfumes, flow-control agents (e.g., anticaking agents), thickeners and the like and mixtures thereof can be used in suitable quantities in the compositions of the invention, as is well known in the art. Suitable excipients and their concentrations in an antiperspirant product are described in U.S. 4,781,917 to Leubbe et al, U.S. 4,774,079 to Shin et al and U.S. 4,083,956 to Shelton.

The antiperspirant products of the invention may be solid, semisolid or liquid in form. When desired, they may be made into gels, pastes, creams, foams, or aerosol liquids using suitable amounts of diluents or other extenders. The formulations are contemplated for use in deodorant/antiperspirant products which are applied as sticks, sprays, roll-ons, creams, lotions, and the like.

The following examples illustrate the invention.

Example 1: Preparation of ZAG salt (Component I)

This example illustrates the preparation of ZAG salts. Solution A below was prepared by admixing the recited ingredients.

Solution A

| Ingredient | (w/w%) |
|---|---|
| ZrO(OH)Cl (50% aqueous solution) | 66.8 |
| AlCl$_3$ (50% aqueous solution) | 19.4 |
| Glycine | 13.8 |
| | 100.00 |

Solution A was heated at about 82°C(180°F) with gentle stirring over a steam bath until a highly thickened, aqueous residue remained, after which the residue was placed in an oven until a powdery solid was obtained.

All dilute solutions used contained water as the diluent. The resultant dried ZAG ingredient (Component I) powder was analyzed as follows.

| Ingredient | Theoretical Amount | Assay Amount | % of Theoretical |
|---|---|---|---|
| Zirconium | 25.5 | 26.6 | 104 |
| Aluminum | 1.9 | 2.0 | 105 |
| Chloride | 17.5 | 18.1 | 103 |

Example 2

The ZAG salt of Example 1 was dry-mixed with an ACH powder obtained from Reheis (RE101) to provide an antiperspirant composition containing 48% ZAG salt and 52% ACH. The ACH was characterized as having a $K_d$ 0.4 concentration of 59%. The dry-mixing was conducted in a twin shell blender for about one hour. This resulted in the final ZAG/ACH antiperspirant composition.

Example 3

Solution A (from Example 1) was spray-dried to obtain the ZAG salt (Component I) in lieu of oven-drying. The resulting ZAG was dry-blended with ACH using the procedure of Example 2 to obtain a final ZAG/ACH antiperspirant composition. Analytical results on these systems were similar to those obtained in Example 1. Spray-drying is the preferred method used to prepare ZAG ingredient (I) powders, rather than evaporation using a steam bath or oven-drying.

The antiperspirant composition was incorporated into a vehicle, the antiperspirant product containing the following ingredients:

| Antiperspirant Product | Wt. % |
|---|---|
| Antiperspirant composition | 24.00 |
| Cyclomethicone 7158 | 53.05 |
| Stearyl alcohol | 13.00 |
| Hydrogenated castor oil | 4.00 |
| PEG-25 Propylene glycol stearate | 1.00 |
| Glyceryl monostearate | 0.35 |
| Butylated hydroxytholuene | 0.05 |
| Talc | 4.00 |
| Fumed silica | 0.50 |
| Pentadecalactone | 0.05 |
| | 100.00 |

Three clinical studies comparing this ZAG/ACH antiperspirant product against Dow Corning 369 (normal tetra ZAG salt) in BAN[R] Solid were conducted. The tests showed that the combination of this invention was 9.7% drier than Dow Corning 369 in the first study, 6.9% drier in the second study, and 8.6% drier in the third study.

Example 4 (Comparative)

100 g of solution A (Example 1) was mixed with 61.66 g of RE101 ACH powder, heated for 90 minutes on a steam bath and dried in an oven as in Example 1. The resultant powder had a $K_d$ 0.4 value of 17%, which is unacceptably low.

Example 5

The antiperspirant composition was prepared by the alternate process of the present invention, as follows:

66.8 g of a 50% aqueous $ZrO(OH)Cl$ solution was mixed with 19.4 g of a 50% aqueous aluminum chloride solution and 13.8 g glycine at room temperature. This solution, which had a ZAG material concentration of 13.8%, was combined with 100 g of a 15.0% aqueous solution of activated ACH in a common vessel. Immediately, the combined solutions were spray-dried to produce a ZAG/ACH antiperspirant composition in powder form.

The concentrations of ingredients in the ZAG and ACH solutions can be adjusted to produce the final ZAG/ACH ratios discussed above under "Antiperspirant (ZAG/ACH) Combination Compositions."

**Claims**

1. A zirconium-aluminum-amino acid material containing on a weight basis: 23.0-34.7wt.% Zr, 1.7-2.5wt.% Al, 21.3-31.9wt.% amino acid and 15.5-23.3wt.% chloride, said material being bound with zero to nine moles water per mole of the material, and obtained by a process comprising the steps of

admixing zirconium hydroxychloride, an aluminum chloro species of the formula $Al_2(OH)_{6-x}Cl_x$ wherein x = 1.5 to 6 and an amino acid in an aqueous solution at from 21 to 116°C (70 to 240°F), and

drying the aqueous solution.

2. A zirconium-aluminum-amino acid material as claimed in claim 1 characterised in that the weight percents of Zr, Al, amino acid and chloride are 25.8-31.6% Zr, 1.9-2.3% Al, 23.9-29.2% amino acid, and 17.5-21.3% chloride.

3. A zirconium-aluminum-amino acid material as claimed in claim 1 or claim 2 characterised in that the amino acid is glycine.

4. A zirconium-aluminum-amino acid material as claimed in claim 1 having empirical formula

$$AlZr_4O_4(OH)_4Cl_7Gly_{4.5} \cdot nH_2O$$

wherein n = 0 to 9.

5. A process for producing a zirconium-aluminum-amino acid material containing on a weight basis: 23.0-34.7 wt.% Zr, 1.7-2.5 wt.% Al, 21.3-31.9 wt.% amino acid and 15.5-23.3 wt.% chloride, said material being bound with zero to nine moles water per mole of the material, said process comprising the steps of

(a) admixing zirconium hydroxychloride, an aluminum chloro species of the formula $Al_2(OH)_{6-x}Cl_x$ wherein x = 1.5 to 6 and an amino acid in an aqueous solution at from 21 to 116°C (70 to 240°F), and

drying the aqueous solution.

6. A process as claimed in claim 5 characterised in that the aqueous solution initially contains on a weight basis from 25 to 40wt.% zirconium hydroxychloride, from 8 to 12wt.% aluminum chloro species and from 11 to 17wt.% amino acid.

7. A process as claimed in claim 5 or claim 6 characterised in that step (a) is effected at a temperature of from 21 to 60°C (70 to 140°F).

8. A process as claimed in claim 5, claim 6 or claim 7 characterised in that the mixing is effected for a period not exceeding 15 minutes.

9. A process as claimed in any one of claims 5 to 8 characterised in that the aluminum chloro species is selected from $AlCl_3$, $Al_2(OH)_4Cl_2$ and $Al_2(OH)_{4.5}Cl_{1.5}$ and the amino acid is glycine.

10. A process as claimed in claim 9 characterised in that the dry ZAG material contains 28.7wt.% Zr, 2.1wt.% Al, 26.6wt.% glycine and 19.4wt.% chloride.

11. A process as claimed in any one of claims 5 to 10 characterised in that in step (b) the aqueous solution is spray-dried.

12. A solid antiperspirant composition comprising a zirconium-aluminum-amino acid (ZAG) material as claimed in any one of claims 1 to 4, or obtained by a method as claimed in any one of claims 5 to 11 and an activated aluminum chlorhydroxide, more than 40% of the Al peaks as determined by gel chromatography of the antiperspirant composition having a $K_d$ value of 0.4.

13. A composition as claimed in claim 12 characterised in that the weight ratio of the ZAG material to the activated aluminum chlorhydroxide is from 2:1 to 1:2.

14. A composition as claimed in claim 12 characterised in that the weight ratio of the ZAG material to the activated aluminum chlorhydroxide is from 1.5:1 to 1:1.5.

15. A process for making an antiperspirant composition as claimed in any one of claims 12 to 14, the process comprising the step of mixing, in a dry state, said ZAG material and said activated aluminum chlorhydroxide.

16. A process for making an antiperspirant composition comprising a zirconium-aluminum-amino acid material containing on a weight basis: 23.0-34.7wt.% Zr, 1.7-2.5wt.% Al, 21.3-31.9wt.% amino acid and 15.5-23.3wt.% chloride, said material being bound with zero to nine moles water per mole of the material, and an activated aluminum chlorhydroxide, more than 40% of the Al peaks as determined by gel chromatography of the antiperspirant composition having a $K_d$ value of 0.4, the process comprising the steps of

(a) admixing zirconium hydroxychloride, an aluminum chloro species of the formula $Al_2(OH)_{6-x}Cl_x$ wherein x = 1.5 to 6 and an amino acid in an aqueous solution at a temperature of less than 52°C (125°F).

(b) admixing an activated aluminum chlorhydroxide with the solution so obtained, and

(c) rapidly drying the mixture.

17. A process as claimed in claim 16 characterised in that step (a) is effected at about room temperature and the admixture is dried by spray-drying.

18. A process as claimed in claim 16 or claim 17 characterised in that step (a) is effected for a period not exceeding 5 minutes.

19. A process as claimed in claim 16, claim 17 or claim 18 characterised in that steps (a) and (c) are completed within 15 minutes.

20. An antiperspirant product comprising an antiperspirant composition as claimed in any one of claims 12 to 14, or obtained by a process as claimed in any one of claims 15 to 19, in a cosmetically acceptable carrier or vehicle.

**Patentansprüche**

1. Zirkonium-Aluminium-Aminosäure-Material, enthaltend (gewichtsbezogen): 23,0-34,7 Gew.-% Zr, 1,7-2,5 Gew.-% Al, 21,3-31,9 Gew.-% Aminosäure und 15,5-23,3 Gew.-% Chlorid, wobei das Material 0 bis 9 Mol Wasser pro Mol des Materials gebunden enthält und durch ein Verfahren erhältlich ist, das folgende Stufen umfaßt:

Vermischen von Zirkoniumhydroxychlorid, einer Aluminiumchlor-Spezies der Formel $Al_2(OH)_{6-x}Cl_x$, worin x = 1,5 bis 6, und einer Aminosäure bei 21 bis 116°C (70 bis 240°F) in einer wäßrigen Lösung und

Trocknen der wäßrigen Lösung.

2. Zirkonium-Aluminium-Aminosäure-Material nach Anspruch 1, dadurch gekennzeichnet, daß Zr, Al, Aminosäure und Chlorid in folgenden Mengen (Gew.-%) vorliegen: 25,8-31,6 % Zr, 1,9-2,3 % Al, 23,9-29,2 % Aminosäure und 17,5-21,3 % Chlorid.

3. Zirkonium-Aluminium-Aminosäure-Material nach Anspruch 1 oder 2, dadurch gekennzeichnet, daß die Aminosäure Glycin ist.

4. Zirkonium-Aluminium-Aminosäure-Material nach Anspruch 1 der Summenformel

$$AlZr_4O_4(OH)_4Cl_7Gly_{4.5} \cdot nH_2O,$$

worin n = 0 bis 9.

5. Verfahren zur Herstellung eines Zirkonium-Aluminium-Aminosäure-Materials, enthaltend (gewichtsbezogen): 23,0-34,7 Gew.-% Zr, 1,7-2,5 Gew.-% Al, 21,3-31,9 Gew.-% Aminosäure und 15,5-23,3 Gew.-% Chlorid, wobei das Material 0 bis 9 Mol pro Mol Wasser des Materials gebunden enthält und das Verfahren folgende Stufen umfaßt:

(a) Vermischen von Zirkoniumhydroxychlorid, einer Aluminiumchlor-Spezies der Formel $Al_2(OH)_{6-x}Cl_x$, worin x = 1,5 bis 6, und einer Aminosäure bei 21 bis 116°C (70 bis 240°F) in einer wäßrigen Lösung und

(b) Trocknen der wäßrigen Lösung.

6. Verfahren nach Anspruch 5, dadurch gekennzeichnet, daß die wäßrige Lösung anfänglich enthält (gewichtsbezogen): 25-40 Gew.-% Zirkoniumhydroxychlorid, 8 bis 12 Gew.-% einer Aluminiumchlor-Spezies und 11 bis 17 Gew.-% Aminosäure.

7. Verfahren nach Anspruch 5 oder 6, dadurch gekennzeichnet, daß Stufe (a) bei einer Temperatur von 21 bis 60°C (70 bis 140°F) durchgeführt wird.

8. Verfahren nach Anspruch 5, 6 oder 7, dadurch gekennzeichnet, daS das Mischen über einen Zeitraum von nicht mehr als 15 Minuten erfolgt.

9. Verfahren nach einem der Ansprüche 5 bis 8, dadurch gekennzeichnet, daß die Aluminiumchlor-Spezies ausgewählt ist unter $AlCl_3$, $Al_2(OH)_4Cl_2$ und $Al_2(OH)_{4.5}Cl_{1.5}$ und daß es sich bei der Aminosäure um Glycin handelt.

10. Verfahren nach Anspruch 9, dadurch gekennzeichnet, daß das trockene ZAG-Material 28,7 Gew.-% Zr, 2,1 Gew.-% Al, 26,6 Gew.-% Glycin und 19,4 Gew.-% Chlorid enthält.

11. Verfahren nach einem der Ansprüche 5 bis 10, dadurch gekennzeichnet, daß die wäßrige Lösung in Stufe (b) sprühgetrocknet wird.

12. Feste Antiperspiranszusammensetzung, umfassend ein Zirkonium-Aluminium-Aminosäure (ZAG)-Material nach einem der Ansprüche 1 bis 4 oder erhältlich gemäß einem Verfahren nach einem der Ansprüche 5 bis 11, und aktiviertes Aluminiumchlorhydroxid, wobei mehr als 40 % der durch Gelchromatographie der Antiperspiranszusammensetzung bestimmten Al-Peaks einen $K_d$-Wert von 0,4 besitzen.

13. Zusammensetzung nach Anspruch 12, dadurch gekennzeichnet, daß das Gewichtsverhältnis des ZAG-Materials zu dem aktivierten Aluminiumchlorhydroxid 2:1 bis 1:2 beträgt.

14. Zusammensetzung nach Anspruch 12, dadurch gekennzeichnet, daß das Gewichtsverhältnis des ZAG-Materials zu dem aktivierten Aluminumchlorhydroxid 1,5:1 bis 1:1,5 beträgt.

15. Verfahren zur Herstellung einer Antiperspirans-Zusammensetzung nach einem der Ansprüche 12 bis 14, wobei das Verfahren eine Stufe umfaßt, bei der man das ZAG-Material und das aktivierte Aluminiumchlorhydroxid in trockenem Zustand vermischt.

16. Verfahren zur Herstellung einer Antiperspirans-Zusammensetzung, umfassend ein Zirkonium-Aluminium-Aminosäure-Material, das enthält (gewichtsbezogen): 23,0-34,7 Gew.-% Zr, 1,7-2,5 Gew.-% Al, 21,3-31,9 Gew.-% Aminosäure und 15,5-23,3 Gew.-% Chlorid, wobei das Material 0 bis 9 Mol Wasser pro Mol des Materials gebunden enthält,
und aktiviertes Aluminiumchlorhydroxid, wobei mehr als 40 % der durch Gelchromatographie der Antiperspiranszusammensetzung bestimmten Al-Peaks einen $K_d$-Wert von 0,4 besitzen,
wobei das Verfahren folgende Stufen umfaßt:

(a) Vermischen von Zirkoniumhydroxychlorid, einer Aluminiumchlor-Spezies der Formel $Al_2(OH)_{6-x}Cl_x$, worin x = 1,5 bis 6, und einer Aminosäure bei einer Temperatur von weniger als 52°C (125°F) in einer wäßrigen Lösung,

(b) Zumischen von aktiviertem Aluminiumchlorhydroxid zu der so erhaltenen Lösung und

(c) rasches Trocknen des Gemisches.

17. Verfahren nach Anspruch 16, dadurch gekennzeichnet, daß Stufe (a) bei etwa Raumtemperatur durchgeführt wird und daß das Gemisch durch Sprühtrocknen getrocknet wird.

18. Verfahren nach Anspruch 16 oder 17, dadurch gekennzeichnet, daß Stufe (a) über einen Zeitraum von nicht mehr als 5 Minuten erfolgt.

19. Verfahren nach Anspruch 16, 17 oder 18, dadurch gekennzeichnet, daß die Stufen (a) und (c) innerhalb von 15 Minuten abgeschlossen sind.

**20.** Antiperspirans-Produkt, umfassend eine Antiperspirans-Zusammensetzung nach einem der Ansprüche 12 bis 14 oder erhältlich gemäß einem Verfahren nach einem der Ansprüche 15 bis 19, in einem kosmetisch verträglichen Träger oder Vehikel.

## Revendications

**1.** Matériau zirconium-aluminium-acide aminé contenant sur une base en poids :

23,0 à 34,7% en poids de Zr, 1,7 à 2,5% en poids d'Al, 21,3 à 31,9% en poids d'acide aminé et 15,5 à 23,3% en poids de chlorure, ledit matériau étant lié avec zéro à neuf moles d'eau par mole du matériau, et obtenu par un procédé comprenant les étapes de
mélange d'hydroxychlorure de zirconium, d'une espèce chloro aluminium de la formule $Al_2(OH)_{6-x}Cl_x$ où x = 1,5 à 6 et d'un acide aminé en solution aqueuse à une température de 21 à 116°C (70 à 240°F), et séchage de la solution aqueuse.

**2.** Matériau zirconium-aluminium-acide aminé selon la revendication 1 caractérisé en ce que les pour cents en poids de Zr, Al, acide aminé et chlorure sont 25,8 à 31,6% de Zr, 1,9 à 2,3% d'Al, 23,9 à 29,2% d'acide aminé, et 17,5 à 21,3% de chlorure.

**3.** Matériau de zirconium-aluminium-acide aminé selon la revendication 1 ou la revendication 2 caractérisé en ce que l'acide aminé est la glycine.

**4.** Matériau zirconium-aluminium-acide aminé selon la revendication 1 ayant la formule empirique

$$AlZr_4O_4(OH)_4Cl_7Gly_{4,5}nH_2O \text{ où } n = 0 \text{ à } 9.$$

**5.** Procédé pour produire un matériau zirconium-aluminium-acide aminé contenant sur une base en poids : 23,0 à 34,7% en poids de Zr, 1,7 à 2,5% en poids d'Al, 21,3 à 31,9% en poids d'acide aminé et 15,5 à 23,3% en poids de chlorure, ledit matériau étant lié avec zéro à neuf moles d'eau par mole de matériau, ledit procédé comprenant les étapes de :

(a) mélange d'hydroxychlorure de zirconium, d'une espèce chloro aluminium de la formule $Al_2(OH)_{6-x}Cl_x$ où x = 1,5 à 6 et d'un acide aminé dans une solution aqueuse à une température de 21 à 116°C (70 à 240°F), et séchage de la solution aqueuse.

**6.** Procédé selon la revendication 5 caractérisé en ce que la solution aqueuse contient initialement sur une base en poids de 25 à 40% en poids d'hydroxychorure de zirconium, de 8 à 12% en poids d'espèce chloro aluminium et de 11 à 17% en poids d'acide aminé.

**7.** Procédé selon la revendication 5, ou la revendication 6 caractérisé en ce que l'étape (a) est effectuée à une température de 21 à 60°C (70 à 140°F).

**8.** Procédé selon la revendication 5, la revendication 6 ou la revendication 7 caractérisé en ce que le mélange est effectué pendant une période n'excédant pas 15 minutes.

**9.** Procédé selon l'une quelconque des revendications 5 à 8, caractérisé en ce que l'espèce chloro aluminium est choisie parmi $AlCl_3$, $Al_2(OH)_4Cl_2$ et $Al_2(OH)_{4,5}Cl_{1,5}$ et l'acide aminé est la glycine.

**10.** Procédé selon la revendication 9 caractérisé en ce que le matériau ZAG sec contient 28,7% en poids de Zr, 2,1% en poids d'Al, 26,6% en poids de glycine et 19,4% en poids de chlorure.

**11.** Procédé selon l'une quelconque des revendications 5 à 10 caractérisé en ce que dans l'étape (b) la solution aqueuse est séchée par vaporisation.

**12.** Composition antiperspirante solide comprenant un matériau zirconium-aluminium-acide aminé (ZAG) selon l'une quelconque des revendications 1 à 4, ou obtenue par une méthode selon l'une quelconque des revendications 5 à 11 et un hydroxychlorure d'aluminium activé, plus de 40% des pics d'Al comme déterminés par chromatographie sur gel de la composition antiperspirante ayant une valeur $K_d$ de 0,4.

**13.** Composition selon la revendication 12 caractérisée en ce que le rapport en poids du matériau ZAG à l'hydroxychlorure d'alumium activé est de 2:1 à 1:2.

**14.** Composition selon la revendication 12 caractérisée en ce que le rapport en poids du matériau ZAG à l'hydroxychlorure d'aluminium activé est de 1,5:1 à 1:1,5.

**15.** Procédé pour fabriquer une composition antiperspirante selon l'une quelconque des revendications 12 à 14, le procédé comprenant l'étape de mélange, à l'état sec, dudit matériau ZAG et dudit hydroxychlorure d'aluminium activé.

**16.** Procédé pour fabriquer une composition antiperspirante comprenant un matériau zirconium-aluminium-acide aminé contenant sur une base en poids : 23,0 à 34,7% en poids de Zr, 1,7 à 2,5% en poids d'Al, 21,3 à 31,9% en poids d'acide aminé et 15,5 à 23,3% en poids de chlorure, ledit matériau étant lié avec zéro à neuf moles d'eau par mole de matériau et un hydroxychlorure d'aluminium activé, plus de 40% des pics d'Al comme déterminés par chromatographie sur gel de la composition antiperspirante ayant une valeur $K_d$ de 0,4, le procédé comprenant les étapes de

   (a) mélange d'hydroxychlorure de zirconium, d'une espèce chloro aluminium de la formule $Al_2(OH)_{6-x}Cl_x$ où x = 1,5 à 6 et d'un acide aminé dans une solution aqueuse à une température inférieure à 52°C(125°F).
   (b) mélange d'un hydroxychlorure d'aluminium avec la solution ainsi obtenue, et
   (c) séchage rapidement du mélange.

**17.** Procédé selon la revendication 16 caractérisé en ce que l'étape (a) est effectuée à environ la température ambiante et en ce que le mélange est séché par séchage par vaporisation.

**18.** Procédé selon la revendication 16 ou la revendication 17 caractérisé en ce que l'étape (a) est effectuée pendant une période n'excédant pas 5 minutes.

**19.** Procédé selon la revendication 16, la revendication 17 ou la revendication 18 caractérisé en ce que les étapes (a) et (c) sont complétées en 15 minutes.

**20.** Produit antiperspirant comprenant une composition antiperspirante selon l'une quelconque des revendications 12 à 14, ou obtenu par un procédé selon l'une quelconque des revendications 15 à 19, dans un véhicule porteur cosmétiquement acceptable.